# EUROPÄISCHE PATENTSCHRIFT

(11) **EP 0 472 553 B1**
(45) Veröffentlichungstag und Bekanntmachung des Hinweises auf die Patenterteilung: **18.01.1995**
(21) Anmeldenummer: 90906978.3
(22) Anmeldetag: 10.05.1990
(51) Int. Cl.: C07C 231/02, C07C 233/36, C07C 67/08, C07C 69/28, C07C 69/30, C08K 5/20, C08K 5/10, C07C 51/41, C07C 53/126

(54) **GEMISCHE AUS FETTSÄUREALKYLENDIAMIDEN, FETTSÄUREESTERN UND METALLSEIFEN, IHRE VERWENDUNG ALS KUNSTSTOFFADDITIVE**
MIXTURES OF FATTY ACID ALKYLENE DIAMIDES, FATTY ACID ESTERS AND METAL SOAPS, AND THEIR USE AS ADDITIVES IN PLASTICS
MELANGES DE ALKYLENEDIAMIDES D'ACIDES GRAS, D'ESTERS D'ACIDES GRAS ET DE SAVONS METALLIQUES; ET LEUR UTILISATION EN TANT QU'ADDITIFS POUR MATIERES SYNTHETIQUES

(30) Priorität: 19.05.1989 DE 3916356
(43) Veröffentlichungstag der Anmeldung: 04.03.1992
(73) Patentinhaber: NEYNABER CHEMIE GmbH, D-27608 Loxstedt (DE)
(72) Erfinder: WORSCHECH, Kurt, D-2854 Loxstedt (DE); FLEISCHER, Erwin, D-2850 Bremerhaven-Spaden (DE); WEDL, Peter, D-2850 Bremerhaven (DE); LÖFFELHOLZ, Frido, D-2850 Bremerhaven-Surheide (DE)
(86) Internationale Anmeldenummer: EP9000754
(87) Internationale Veröffentlichungsnummer: WO9014332

(56) Entgegenhaltungen:
- EP-A- 0 273 283
- DE-A- 1 952 912
- DE-A- 3 817 924
- US-A- 3 122 504
- US-A- 3 661 824

## Beschreibung

Die Erfindung betrifft ein Verfahren zur Herstellung von Gemischen aus hellfarbigen bis(C₈-C₂₂-Fettsäure)alkylendiamiden und C₈-C₂₂-Fettsäureestern sowie gegebenenfalls Metallseifen von C₈-C₂₂-Fettsäuren.

Bis(C₈-C₁₈-Fettsäure)alkylendiamide, z.B. Ethylendiamindistearat, werden als Additive in der Kunststoffindustrie eingesetzt. Sie dienen z.B. bei der Verarbeitung harter und weicher PVC-Massen als Gleitmittel und verleihen den Fertigprodukten, z.B. Folien, ausgezeichnete Antiblockeigenschaften. Sie eignen sich jedoch auch als Gleit- und Trennmittel für Kunststoffe auf Basis von Polystyrol. Auch in anderen Thermoplasten, z.B. bei der Verarbeitung von Polyestern und Polyamiden, werden sie eingesetzt. Im Vergleich zu anderen Gleit- und Trennmitteln weisen sie hohe Schmelzpunkte (ca. 140°C) auf. Sie haben daher keinen negativen Einfluß auf die Wärmeformbeständigkeit der mit ihnen ausgerüsteten Kunststoffe. Aufgrund ihrer physiologisch günstig zu beurteilenden Eigenschaften werden sie für die Herstellung von PVC-Massen für Lebensmittelverpackungen, Pharmaverpackungen, Spielsachen und medizinischen Schläuchen herangezogen.

Die Ausgangsprodukte der bis(C₈-C₂₂-Fettsäure)alkylendiamide, im folgenden mit Fettsäurealkylendiamide bezeichnet, z.B. Ethylendiamin oder Hexamethylendiamin, sind bei den für die Umsetzung mit Fettsäuren erforderlichen Reaktionstemperaturen (ca. 170°C) sehr oxidationsempfindlich; zweckmäßigerweise erfolgt daher die Umsetzung mit Fettsäuren unter Schutzgas bzw. Ausschluß von Luft bzw. Sauerstoff. Ähnlich empfindlich sind auch die Fettsäurealkylendiamide bei ihrer Konfektionierung durch Sprühen oder Schuppen. Dabei kommt es normalerweise, d.h. in Gegenwart von Luft bzw. Sauerstoff, zu Verfärbungen, so daß braungefärbte Endprodukte erhalten werden. Man kann Fettsäurealkylendiamide zwar auch durch Mahlen in geeignete konfektionierte Produkte kleiner Teilchengröße überführen, dies ist jedoch eine arbeitsintensive und teure Maßnahme.

Aus den vorgenannten Gründen sind für die Herstellung von Fettsäurealkylendiamiden Spezialanlagen erforderlich. Ihre Herstellung in üblichen Industrieanlagen (Veresterungsanlagen, Sprühtürmen) ist in ausreichender Qualität nicht durchführbar.

In der DE-A-38 17 924 wird eine Acetal-Harzmasse beschrieben, die 100 Gew.-Teile eines Acetalharzes und 0,01 bis 2 Gew.-Teile eines Fettsäureesters enthält. Diese Fettsäureester basieren auf Fettsäuren mit 22 bis 32 C-Atomen und mehrwertigen Alkoholen mit 2 bis 10 C-Atomen. Zusätzlich können die Harzmassen gegebenenfalls noch 0,01 bis 2 Gew.-Teile eines Amids einer linearen Fettsäure mit mindestens 10 C-Atomen enthalten.

Die US-A-3 661 824 betrifft Antiblocking-Additive für Acrylatfilme, bestehend aus einer Kombination von Ethylen-N-bis-ölsäureamid und einem speziellen Glyceridester von Myristinsäure, Palmitinsäure und Stearinsäure. Gegenstand der DE-A-19 52 912 sind glasklare, transparente Formkörper aus Polyamid, die als Additive 0,05 bis 3 Gew.-% an Estern oder Amiden von linearen Alkanmonocarbonsäuren mit 10 bis 20 C-Atomen und aliphatischen Alkoholen mit 1 bis 12 C-Atomen oder aliphatischen Diaminen mit 2 bis 12 C-Atomen enthalten.

Ferner ist aus der US-A-3 122 504 ein Verfahren zur Herstellung von partiell amidierten Kondensationsprodukten aus Polyalkylen-polyaminen oder Alkylendiaminen und Estern höherer Fettsäuren bekannt. Die Umsetzung verläuft bei Temperaturen unterhalb von 100 °C, bei kurzen Reaktionszeiten in saurem Milieu. Die entstandenen Reaktionsprodukte, die als Textilhilfsmittel Verwendung finden, bestehen hauptsächlich aus Gemischen von nicht umgesetzten Fettsäureestern und Fettsäuremonoamiden, wobei die freien Aminogruppen der Monoamide mit kurzkettigen Monocarbonsäuren neutralisiert sind.

Demgegenüber beruht die vorliegende Erfindung auf der Erkenntnis, daß sich die vorstehend genannten Nachteile der Oxidationsempfindlichkeit von Alkylendiaminen und Fettsäurealkylendiamiden vermeiden lassen, wenn man Gemische aus Fettsäurealkylendiamiden, Fettsäureestern und gegebenenfalls Metallseifen von Fettsäuren in einer die Fettsäure enthaltenden Schmelze herstellt. Die in den Gemischen enthaltenen Fettsäurealkylendiamide sind hinsichtlich ihrer Wirkung als Additive für Kunststoffe den reinen Fettsäurealkylendiamiden gleichwertig.

Demgemäß ist die Erfindung auf ein Verfahren zur Herstellung von Gemischen aus hellfarbigen bis(C₈-C₂₂-Fettsäure)alkylendiamiden und C₈-C₂₂-Fettsäureestern sowie gegebenenfalls Metallseifen von C₈-C₂₂-Fettsäuren gerichtet, bei dem man in einer Fettsäuren mit 8 bis 22 Kohlenstoffatomen enthaltenden Schmelze einen Teil der Fettsäuren mit Diaminen der allgemeinen Formel

NH₂-R-NH₂

in der R eine geradkettige, verzweigte oder cyclische Alkylengruppe mit 2 bis 12 Kohlenstoffatomen ist, zu den entsprechenden Fettsäurediamiden kondensiert, einen Teil der Fettsäuren mit Fettalkoholen mit 8 bis 22 Kohlenstoffatomen und/oder mehrfunktionellen Alkanolen mit 2 bis 15 Kohlenstoffatomen und 2 bis 6 Hydroxylgruppen verestert sowie gegebenenfalls einen Teil der Fettsäuren mit basischen Verbindungen zweiwertiger Metalle zu den entsprechenden Metallseifen umsetzt, wobei man die Kondensation der Fettsäuren mit den Diaminen vor der Veresterung der Fettsäuren mit den Fettalkoholen und/oder mehrfunktionellen Alkanolen durchführt.

Einer der Vorteile des Verfahrens der Erfindung liegt darin begründet, daß die Amidierung in einem Überschuß von Fettsäure erfolgt. Dabei wird die für die Amidierung und Veresterung sowie gegebenenfalls die Metallseifenbildung erforderliche Fettsäure in Form einer Schmelze eingesetzt. Dies hat den Vorteil, daß auch bei fortschreitender Dauer der Amidierung eine rasche Umsetzung erfolgt, wobei die geschmolzene Säure als Verdünnungsmittel und Schutzmedium für die Fettsäurealkylendiamide dient. Es gelingt weiterhin, die Amidierung sehr schnell zum Ende zu führen, so daß kein freies Amin im Reaktionsgemisch zurückbleibt. Dennoch erfolgt zweckmäßigerweise die Amidierungsstufe unter Schutzgas. Dagegen kann die Veresterungsstufe und die Seifenbildung unter Normalbedingungen, d.h. ohne Schutzgas, erfolgen. Das Reaktionsgemisch kann ohne weiteres unter Standardbedingungen versprüht und geschuppt werden; die vorhandenen Fettsäureester und gegebenenfalls die Metallseifen schützen dabei das gebildete Amidwachs vor Reaktionen mit Luftsauerstoff.

Als C₈-C₂₂-Fettsäuren eignen sich im Verfahren der Erfindung solche synthetischen oder insbesondere natürlichen Ursprungs, wie sie z.B. aus pflanzlichen und tierischen Fetten und Ölen, insbesondere Palmöl, Palmkernöl, Kokosöl, Sojaöl, Rüböl, Sonnenblumenöl, Rindertalg und Schweineschmalz erhalten werden können. Wie in der Fettchemie üblich, werden derartige Fettsäuren meist in Form ihrer technischen Gemische eingesetzt. Typische Beispiele für derartige Fettsäuren sind Capryl-, Caprin-, Laurin-, Myristin-, Palmitin-, Stearin-, Arachidin- und Behensäure. Bevorzugt werden gesättigte Fettsäuren mit 16 bis 22 Kohlenstoffatomen einschließlich technische Gemische derselben aus der obigen Aufstellung verwendet.

Typische Beispiele für erfindungsgemäß einzusetzende Diamine sind Ethylendiamin, Propylendiamine, Butylendiamine, Hexylendiamine, Diaminocyclohexan, bis-Methylamino-cyclohexan, Octylendiamine, Decylendiamine und Dodecylendiamine; bevorzugt sind Ethylendiamin, Tetramethylendiamin und Hexamethylendiamin.

Die im Verfahren der Erfindung einsetzbaren Fettalkohole mit 8 bis 22 Kohlenstoffatomen können von den obengenannten Fettsäuren abgeleitet sein; Typische Beispiele für diese Fettalkohole sind Capryl-, Caprin-, Lauryl-, Myristyl-, Cetyl-, Stearyl-, Arachidyl- und Behenylalkohol einschließlich technische Gemische derselben. Bevorzugt sind hier gesättigte Fettalkohole mit 12 bis 22 Kohlenstoffatomen, da die mit diesen erhaltenen Gemische der Erfindung auch bei höheren Temperaturen fest sind.

Anstelle der oder zusammen mit den vorgenannten Fettalkoholen können in dem Verfahren der Erfindung auch mehrfunktionelle Alkanole mit 2 bis 15, insbesondere 2 bis 12 Kohlenstoffatomen und 2 bis 6 Hydroxylgruppen eingesetzt werden. Bevorzugte Beispiele für derartige mehrfunktionelle Alkanole sind Ethylenglykol, Propylenglykol, Glycerin, Diglycerin, Triglycerin, Tetraglycerin, Trimethylolpropan, Di-trimethylolpropan, Pentaerythrit und Di-pentaerythrit; der Einsatz von anderen mehrfunktionellen Alkanolen wie Neopentylglykol, Tri-pentaerythrit und dergleichen ist jedoch ebenfalls möglich. Die mehrfunktionellen Alkanole werden erfindungsgemäß mit den Fettsäuren zu Voll- oder Partialestern umgesetzt.

Als in dem Verfahren der Erfindung einsetzbare basische Verbindungen zweiwertiger Metalle sind insbesondere die Oxide, Hydroxide und Carbonate von Mg, Ca, Ba, Cd, Zn und Pb zu nennen, wobei Ca und/oder Zn-Basen bevorzugt sind.

Gemäß einer bevorzugten Ausführungsform der Erfindung setzt man bei der Kondensation 2,2 bis 10, insbesondere 2,5 bis 4 Mol der in der Schmelze enthaltenden Fettsäuren pro Mol Diamin ein. Der Überschuß der Fettsäure richtet sich dabei nach dem Gehalt der erfindungsgemäß herzustellenden Gemische an Fettsäureestern und gegebenenfalls Metallseifen, wobei man bestrebt ist, den Fettsäureestergehalt möglichst niedrig zu halten.

Gemäß einer weiteren vorteilhaften Ausführungsform der Erfindung stellt man in der die Fettsäuren enthaltenden Schmelze die Fettsäurediamide, Fettsäureester und gegebenenfalls die Metallseifen in Gewichtsverhältnissen zueinander von 4 bis 6 : 1 bis 2 : 2 bis 4 her.

Die in der Schmelze vorliegende Fettsäure dient in dem Verfahren der Erfindung als Lösemittel, so daß ein Zusatz von inerten Lösemitteln nicht erforderlich ist.

Gemäß einer weiteren vorteilhaften Ausführungsform des Erfindung kondensiert man die Fettsäuren und die Diamine bei Temperaturen der Schmelze von 140 bis 190, insbesondere von 160 bis 180°C. Das dabei freiwerdende Reaktionswasser wird fortlaufend aus dem Reaktionsgemisch entfernt, wobei es vorteilhaft sein kann, die Reaktion unter leichtem Vakuum durchzuführen; entsprechendes gilt auch für die Schritte der Veresterung und der Seifenbildung.

Die Kondensationsreaktion wird bevorzugt beendet, wenn eine Aminzahl von weniger als 1 erreicht ist. Die Aminzahl ist als diejenige Menge an Kaliumhydroxid in Milligramm definiert, die erforderlich ist, um die von 1 g Reaktionsgemisch gebundene Salzsäuremenge zu neutralisieren.

Gemäß einer weiteren vorteilhaften Ausführungsform der Erfindung setzt man die Fettsäuren und Fettalkohole bzw. mehrfunktionellen Alkanole bei Temperaturen der Schmelze von 150 bis 190°C in Gegenwart von Veresterungskatalysatoren um. Hierfür geeignete Veresterungskatalysatoren sind dem Fachmann geläufig; typische Beispiele hierfür sind Zinnkatalysatoren, insbesondere Zinnoxalat.

Gemäß einer weiteren vorteilhaften Ausführungsform der Erfindung nimmt man die Umsetzung der Fettsäuren zu den Metallseifen bei Temperaturen der Schmelze im Bereich von 150 bis 160°C vor. Zweckmäßigerweise wählt man eine Temperatur unterhalb der Reaktionstemperaturen der Amidierung und Veresterung, um Nebenreaktionen zu vermeiden, falls in dem Reaktionsgemisch bereits Fettsäurediamide und Fettsäureester vorhanden sind.

Für die Reihenfolge der Verfahrensstufen der Amidierung, Veresterung und Seifenbildung ist lediglich wichtig, daß die Amidierung vor der Veresterung erfolgt. Dagegen kann die Metallseifenbildung vor und/oder nach der Amidierung und vor und/oder nach der Veresterung erfolgen. Im allgemeinen ist es vorteilhaft, zunächst in der die Fettsäuren enthaltenden Schmelze die Amidierung, danach die Veresterung und anschließend die Seifenbildung vorzunehmen. Wenn jedoch Zinkseifenhaltige Gemische hergestellt werden sollen, kann es bei höheren Zinkgehalten zu Löslichkeitsproblemen kommen. Daher ist es vorteilhaft, bei der Herstellung derartiger Gemische der Fettsäureschmelze vor der Amidierung und Veresterung sowie gegebenenfalls der Metallseifenbildung eine Zinkseife der Fettsäuren zuzusetzen bzw. in dieser zu erzeugen, z.B. durch Eintragung von Zinkoxid. Überraschenderweise treten dabei die vorstehend genannten Löslichkeitsprobleme nicht auf, und es ergeben sich auch keine Nachteile hinsichtlich der nachfolgenden Amidierung und Veresterung. Im Anschluß daran kann man gegebenenenfalls noch vorhandene freie Fettsäuren mit anderen basischen Metallverbindungen als ZnO, z.B. mit CaO, zu den entsprechenden Metallseifen umsetzen, so daß man Mischseifensysteme erhält. Im übrigen kann das hier erläuterte Verfahrensprinzip der Herstellung eines Fettsäure/Metallseifen-Gemisches in der ersten Stufe auch bei sämtlichen anderen der hier in Frage kommenden Metallseifen angewendet werden. Bevorzugt ist jedoch diejenige Variante, gemäß der man in der Fettsäuren und gegebenenfalls Zinkseifen enthaltenden Schmelze nacheinander die Fettsäurediamide und Fettsäureester sowie gegebenenfalls die Metallseifen aus der von Mg, Ca, Ba, Cd und Pb gebildeten Gruppe, insbesondere Ca-Seifen, erzeugt.

Bei der Herstellung von Gemischen der Erfindung, die neben Fettsäurealkylendiamiden und gegebenenfalls Metallseifen Ester von Fettsäuren mit mehrfunktionellen Alkanolen enthalten, kann die Reaktion durch Auswahl geeigneter Ansatzverhältnisse so geführt werden, daß die Ester - jedenfalls rechnerisch - als Partialester oder als Vollester vorliegen. Wenn ein Gemisch mit einem Gehalt an Fettsäurepartialestern hergestellt werden soll, arbeitet man dabei mit einem Überschuß an OH-Gruppen, bezogen auf vorhandene Carboxylgruppen der Fettsäuren. Dabei kann der Anteil an freien Fettsäuren in dem Reaktionsgemisch so stark absinken, daß nicht mehr genügend Fettsäuren für eine eventuell vorzunehmende Seifenbildung zur Verfügung stehen. In diesen Fällen ist es bevorzugt, dem Reaktionsgemisch nach der Amidierung und Veresterung weitere Fettsäuren mit 8 bis 22 Kohlenstoffatomen zuzusetzen, so daß für die Seifenbildung eine ausreichende Menge an Fettsäuren zur Verfügung steht.

Im allgemeinen weisen die erfindungsgemäß erhaltenen Gemische Säurezahlen von weniger als 5 auf, d.h. sie enthalten nur noch geringe oder keine Anteile an freien Fettsäuren. Da ein gewisser Anteil an freien Fettsäuren in den Gemischen jedoch nicht stört und unter Umständen beim Einsatz der Gemische als Additive für Kunststoffmassen auf Basis von PVC sogar vorteilhaft sein kann, können mit dem Verfahren der Erfindung ohne weiteres auch Gemische mit Säurezahlen im Bereich von 5 bis 20 erhalten werden; dies entspricht einem Anteil der freien Fettsäuren am Gesamtgemisch von etwa 3 bis 10 Gew.-%. Derartige, freie Fettsäuren enthaltende Gemische sind insbesondere bei Zinkseifen-haltigen Systemen bevorzugt, da sich diese Systeme in Anwesenheit freier Fettsäuren völlig homogen erhalten lassen.

Die Erfindung betrifft weiterhin Gemische von hellfarbigen bis(C₈-C₂₂-Fettsäure)alkylendiamiden mit Estern von C₈-C₂₂-Fettsäuren mit Fettalkoholen mit 8 bis 22 Kohlenstoffatomen und/oder mehrfunktionellen Alkanolen mit 2 bis 15 Kohlenstoffatomen und 2 bis 6 Hydroxylgruppen und gegebenenfalls Metallseifen von C₈-C₂₂-Fettsäuren in Gewichtsverhältnissen zueinander von 4 bis 6 : 1 bis 2 : 2 bis 4, erhältlich nach einem Verfahren der vorliegenden Erfindung. Schließlich betrifft die Erfindung die Verwendung der vorgenannten, hellfarbige bis(C₈-C₂₂-Fettsäure)alkylendiamide enthaltenden Gemische als Additive für Kunststoffe, insbesondere auf Basis von PVC bzw. Copolymeren derselben.

Die Erfindung wird im folgenden anhand bevorzugter Ausführungsbeispiele näher erläutert.

Beschreibung einiger der in den Ausführungsbeispielen eingesetzten Reagenzien (die Prozentangaben beziehen sich auf Gewichtsprozent).
1. Technische Stearinsäure:
   Handelsübliche Qualität, Säurezahl 206 bis 210, Kettenverteilung: 4 % < C₁₆, 45 % C₁₆, 2 % C₁₇, 47 % C₁₈, 1 % C₁₈ (einfach ungesättigt), 1 % > C₁₈, Jodzahl ca. 1.
2. Laurinsäure:
   Handelsübliche Qualität, Säurezahl 276 bis 280, Kettenverteilung: < 1 % C₁₀, > 93 % C₁₂, 4 bis 6 % C₁₄.
3. Behensäure:
   Handelsübliche Qualität, Säurezahl 164 bis 168, Kettenverteilung: < 2 % C₁₆, < 3 % C₁₈, < 10 % C₂₀, > 80 % C₂₂, < 2 % C₂₄.
4. Talgfettalkohol:
   Handelsübliche Qualität, Hydroxylzahl 215 bis 222, Kettenverteilung: < 2 % C₁₂, 3 bis 7 % C₁₄, 26 bis 33 % C₁₆, 60 bis 65 % C₁₈, < 2 % c₂₀.
5. Hexamethylendiamin (HMDA), Ethylendiamin (EDA):
   handelsübliche Qualität.

### Beispiel 1.

### Gemisch aus Calciumstearat, bis(Stearinsäure)-hexamethylendiamid/Stearinsäure-ethylenglykolester.

728,5 g (2,70 Mol) technische Stearinsäure wurden in einem Reaktor vorgelegt, auf 100°C erhitzt und mit 86 g (0,74 Mol) Hexamethylendiamin versetzt. Die Schmelze wurde unter Schutzgas auf 170°C erhitzt; das Kondensationswasser wurde fortlaufend durch Destillation abgezogen. Nachdem das Reaktionsgemisch eine Aminzahl von weniger als 1 erreicht hatte, wurden 50,4 g (0,81 Mol) Ethylenglykol zugegeben und die Temperatur auf 190°C gesteigert. Die Veresterung erfolgte nach Zugabe von 0,1 g Sn-Oxalat, wobei das Reaktionswasser unter leichtem Vakuum entfernt wurde. Die Reaktion wurde beeendet, nachdem die Säurezahl des, Reaktionsgemisches unter 5 gesunken war.

Zur Bildung der Calciumseife wurden 358,0 g (1,33 Mol) technische Stearinsäure in das Reaktionsgemisch gegeben. Anschließend wurde die Temperatur des Reaktionsgemischs auf 150 bis 155°C eingestellt; danach wurden innerhalb von 15 min portionsweise 49,0 g (0,66 Mol) Calciumhydroxid zugesetzt; das Reaktionswasser wurde im Wasserstrahlvakuum entfernt. Nach einer Reaktionszeit von 2 Stunden hatte sich ein Druck von 22 hPa eingestellt; die Säurezahl war auf 5 gesunken. Die Schmelze wurde vor dem Erstarren in eine Wanne gegossen. Man erhielt das Titelgemisch in Form einer schwach gelbstichigen, wachsartigen Masse mit einem Schmelzpunkt von 130°C und einem Ca-Gehalt von 2,25 %. Das Gemisch enthielt 33 Gew.-% Calciumstearat, 38 Gew.-% bis(Stearinsäure)-hexamethylendiamid und 29 Gew.-% Ethylenglykolmono/distearat.

### Beispiel 2.

### Gemisch aus Zinkstearat, bis(Stearinsäure)-hexamethylendiamid und Stearinsäure-ethylenglykolester.

472,4 g (1,75 Mol) technische Stearinsäure wurden in einem Reaktor vorgelegt, auf 150°C erhitzt und innerhalb von 10 min mit 23,5 g (0,29 Mol) Zinkoxid portionsweise versetzt. Das gebildete Neutralisationswasser wurde im Wasserstrahlvakuum entfernt; innerhalb von 1,5 h hatte sich ein Druck von 30 hPa eingestellt. Anschließend wurden unter Schutzgas 37,4 g (0,32 Mol) Hexamethylendiamin zugegeben; danach wurde die Temperatur auf 170°C erhöht. Das Fortschreiten der Reaktion wurde über die Säurezahl kontrolliert. Nachdem eine Säurezahl von 48 (Theorie: 50) erreicht war, wurden 21,9 g (0,35 Mol) Ethylenglykol und 0,1 g Zinnoxalat zugegeben. Die Reaktion wurde unter schwachem Vakuum bis 450 hPa fortgesetzt, bis eine Säurezahl von 5 erreicht war. Die Reaktionszeit betrug 2,5 h; das Reaktionswasser wurde fortlaufend entfernt. Anschließend wurde die Schmelze abgekühlt und bei 150°C zum Erstarren in eine Wanne gegossen. Es resultierte eine schwach gelbstichige, wachsartige Masse mit einem Schmelzpunkt von 135°C und einem Zn-Gehalt von 3,6 %. Das Produkt wies eine Zusammensetzung von 34 Gew.-% Zinkstearat, 38 Gew.-% bis(Stearinsäure)-hexamethylendiamid und 28 % Ethylenglykol-mono/distearat auf.

### Beispiel 3.

### Gemisch aus bis(Stearinsäure)-hexamethylendiamid und Stearinsäure-ethylenglykolester (Gewichtsverhältnis 1 : 1).

565,8 g (2,1 Mol) technische Stearinsäure (Molmasse 270) wurden unter Rühren auf 100°C erhitzt und unter Schutzgas mit 58 g (0,5 Mol) Hexamethylendiamin versetzt. Die Temperatur wurde auf 170°C gesteigert; das Reaktionswasser wurde fortlaufend entfernt. Die Kondensation wurde nach Erreichen einer Aminzahl von weniger als 1 beendet. Bei der gleichen Temperatur wurden 33,9 g (0,55 Mol) Ethylenglykol und 0,2 g Zinnoxalat hinzugegeben; danach wurde die Temperatur auf 190°C erhöht. Die Reaktion wurde im Vakuum durchgeführt; das Reaktionswasser wurde fortlaufend entfernt. Nachdem der Druck auf 25 hPa abgesunken war, wurde auf 150°C abgekühlt. Die Schmelze wurde zum Erstarren in eine Wanne gegossen. Man erhielt eine gelbstichige, wachsartige Masse mit einem Schmelzpunkt von 127°C.

### Beispiel 4.

### Gemisch aus bis(Stearinsäure)-hexamethylendiamid und Stearinsäure-ethylenglykolester (Gewichtsverhältnis 3 : 1)

891,7 g (3,30 Mol) technische Stearinsäure wurden in einem Reaktor vorgelegt, auf 100°C erhitzt und unter Schutzgas mit 140,3 g (1,21 Mol) Hexamethylendiamin versetzt. Anschließend wurde die Temperatur unter Schutzgas auf 170°C erhöht; die Kondensation wurde nach Erreichen einer Aminzahl von weniger als 1 beendet. Anschließend wurden 27,4 g (0,44 Mol) Ethylenglykol sowie 0,02 Gew.-%, bezogen auf Gesamtansatz, Zinnoxalat hinzugegeben. Die Temperatur wurde auf 190°C gesteigert; die Veresterung wurde im Vakuum durchgeführt. Nach 3 h wurde eine Säurezahl von weniger als 10 erreicht. Die Schmelze wurde abgekühlt und bei 150°C in eine Wanne gegossen. Man erhielt eine gelbstichige, wachsartige Masse mit einem Schmelzpunkt von 135°C.

### Beispiel 5.

### Gemisch aus bis(Stearinsäure)-hexamethylendiamid und Stearinsäure-diglycerid (Gewichtsverhältnis 1 : 1).

559 g (2,06 Mol) technische Stearinsäure wurden unter Rühren auf 100°C erhitzt und mit 46,3 g (0,53 Mol) Ethylendiamin innerhalb von 15 min portionsweise versetzt. Anschließend wurde die Temperatur auf 170°C unter Schutzgas gesteigert. Nach 6 h Reaktionszeit war die Aminzahl auf unter 1 gesunken. Anschließend wurden 36,3 g (0,50 Mol) Glycerin und 0,2 g Zinnoxalat zugegeben. Die Veresterung der freien Stearinsäure wurde im Vakuum bei 190°C durchgeführt, bis eine Säurezahl von unter 5 erreicht war. Nach dem Ausgießen in eine Wanne bei 150°C erhielt man eine schwach gelbliche Masse mit einem Schmelzpunkt von 127°C.

### Beispiel 6.

### Gemisch aus bis(Laurinsäure)-hexamethylendiamid und Laurinsäure-ethylenglykolester (Gewichtsverhältnis 1 : 1).

443,0 g (2,21 Mol) technische Laurinsäure wurden auf 100°C erhitzt und portionsweise mit 60,4 g (0,52 Mol) Hexamethylendiamin unter Schutzgas versetzt. Zur Kondensation wurde die Temperatur auf 170°C gesteigert, bis die Aminzahl auf weniger als 1 abgesunken war. Anschließend wurden 36,4 g (0,585 Mol) Ethylenglykol und 0,2 g Zinnoxalat hinzugegeben; die Veresterung der freien Laurinsäure erfolgte unter Vakuum bei 190°C. Nachdem der Druck bis auf 25 hPa abgesunken war, wurde die Schmelze auf 150°C abgekühlt und in eine Wanne gegossen. Man erhielt eine beigefarbene, wachsartige Masse mit einem Festpunkt von 128°C.

### Beispiel 7.

### Gemisch aus bis(Behensäure)-hexamethylendiamid und Behensäure-ethylenglykolester (Gewichtsverhältnis 1 : 1).

464,5 g (1,37 Mol) Behensäure wurden auf 100°C erhitzt und unter Schutzgas portionsweise mit 38,2 g (0,33 Mol) Hexamethylendiamin versetzt. Anschließend wurde die Temperatur auf 170°C gesteigert, bis eine Aminzahl von weniger als 1 erreicht war. Anschließend wurden 22,0 g (0,355 Mol) Ethylenglykol und 0,2 g Zinnoxalat zugegeben. Die Veresterung erfolgte bei 190°C unter Vakuum. Nachdem der Druck auf 25 hPa gesunken war, wurde das Reaktionsgemisch auf 150°C abgekühlt und in eine Wanne gegossen. Nach dem Erstarren erhielt man eine beigefarbene, wachsartige Masse mit einem Schmelzpunkt von 133,5°C.

### Beispiel 8.

### bis(Stearinsäure)-ethylendiamid und Stearinsäure-talgfettalkoholester (Gewichtsverhältnis 3 : 1).

339,9 g (1,26 Mol) technische Stearinsäure wurden in einem Reaktor vorgelegt, auf 100°C erhitzt und portionsweise unter Schutzgas mit 31,9 g (0,532 Mol) Ethylendiamin innerhalb von 15 min versetzt. Nach einer Reaktionszeit von 6 Stunden bei 170°C war die Aminzahl auf unter 1 gesunken. Anschließend wurden 80 g (0,195 Mol) Talgfettalkohol und 0,1 g Zinnoxalat zugegeben; die Veresterung der freien Stearinsäure erfolgte im Vakuum bei 190°C. Nachdem der Druck auf 25 hPa abgesunken war, wurde das Reaktionsgemisch auf 150°C abgekühlt und in eine Wanne gegossen. Nach dem Erstarren erhielt man eine gelbstichige, wachsartige Masse mit einem Schmelzpunkt von 135,5°C.

## Patentansprüche

1. Verfahren zur Herstellung von Gemischen aus hellfarbigen bis(C₈-C₂₂-Fettsäure)alkylendiamiden und C₈-C₂₂-Fettsäureestern sowie gegebenenfalls Metallseifen von C₈-C₂₂-Fettsäuren, dadurch gekennzeichnet, daß man in einer Fettsäuren mit 8 bis 22 Kohlenstoffatomen enthaltenden Schmelze einen Teil der Fettsäuren mit Diaminen der allgemeinen Formel
NH₂-R-NH₂
in der R eine geradkettige, verzweigte oder cyclische Alkylengruppe mit 2 bis 12 Kohlenstoffatomen ist, zu den entsprechenden Fettsäurediamiden kondensiert, einen Teil der Fettsäuren mit Fettalkoholen mit 8 bis 22 Kohlenstoffatomen und/oder mehrfunktionellen Alkanolen mit 2 bis 15 Kohlenstoffatomen und 2 bis 6 Hydroxylgruppen verestert sowie gegebenenfalls einen Teil der Fettsäuren mit basischen Verbindungen zweiwertiger Metalle zu den entsprechenden Metallseifen umsetzt, wobei man die Kondensation der Fettsäuren mit den Diaminen vor der Veresterung der Fettsäuren mit den Fettalkoholen und/oder mehrfunktionellen Alkanolen durchführt.

2. Verfahren nach Anspruch 1, dadurch gekennzeichnet, daß man gesättigte Fettsäuren mit 16 bis 22 Kohlenstoffatomen oder Gemische derselben verwendet.

3. Verfahren nach Anspruch 1 oder 2, dadurch gekennzeichnet, daß man Diamine aus der von Ethylendiamin, Tetramethylendiamin und Hexamethylendiamin gebildeten Gruppe verwendet.

4. Verfahren nach mindestens einem der Ansprüche 1 bis 3, dadurch gekennzeichnet, daß man bei der Kondensation 2,2 bis 10, insbesondere 2,5 bis 4 Mol der in der Schmelze enthaltenen Fettsäuren pro Mol Diamin einsetzt.

5. Verfahren nach mindestens einem der Ansprüche 1 bis 4, dadurch gekennzeichnet, daß man in der Schmelze die Fettsäurediamide, Fettsäureester und gegebenenfalls die Metallseifen in Gewichtsverhältnissen zueinander von 4 bis 6 : 1 bis 2 : 2 bis 4 herstellt.

6. Verfahren nach mindestens einem der Ansprüche 1 bis 5, dadurch gekennzeichnet, daß man die Fettsäuren und Diamine bei Temperaturen von 140 bis 190, insbesondere 160 bis 180°C, kondensiert.

7. Verfahren nach mindestens einem der Ansprüche 1 bis 6, dadurch gekennzeichnet, daß man die Kondensation bei Erreichen einer Aminzahl des Reaktionsgemisches von weniger als 1 beendet.

8. Verfahren nach mindestens einem der Ansprüche 1 bis 7, dadruch gekennzeichnet, daß man die Fettsäuren und Fettalkohole bzw. mehrfunktionellen Alkanole bei Temperaturen der Schmelze von 150 bis 190°C in Gegenwart von Veresterungskatalysatoren umsetzt.

9. Verfahren nach mindestens einem der Ansprüche 1 bis 8, dadurch gekennzeichnet, daß man für die Veresterung der Fettsäuren gesättigte Fettalkohole mit 12 bis 22 Kohlenstoffatomen verwendet.

10. Verfahren nach mindestens einem der Ansprüche 1 bis 9, dadurch gekennzeichnet, daß man für die Veresterung mehrfunktionelle Alkanole aus der von Ethylenglykol, Propylenglykol, Glycerin, Diglycerin, Triglycerin, Tetraglycerin, Trimethylolpropan, Di-trimethylolpropan, Pentaerythrit und Di-pentaerythrit gebildeten Gruppe verwendet und diese mit den Fettsäuren zu Voll- oder Partialestern umsetzt.

11. Verfahren nach mindestens einem der Ansprüche 1 bis 10, dadurch gekennzeichnet, daß man die Umsetzung zu den Metallseifen bei Temperaturen der Schmelze von 150 bis 160°C vornimmt.

12. Verfahren nach mindestens einem der Ansprüche 1 bis 11, dadurch gekennzeichnet, daß man für die Bildung der Metallseifen der Fettsäuren Oxide, Hydroxide und/oder Carbonate eines Metalls oder mehrerer aus der von Mg, Ca, Ba, Cd, Zn und Pb gebildeten Gruppe, insbesondere Ca und/oder Zn, verwendet.

13. Verfahren nach mindestens einem der Ansprüche 1 bis 12, dadurch gekennzeichnet, daß man der die Fettsäuren enthaltenden Schmelze vor der Kondensation und Veresterung sowie gegebenenfalls der Metallseifenbildung eine Zinkseife der Fettsäuren zusetzt bzw. in dieser erzeugt.

14. Verfahren nach mindestens einem der Ansprüche 1 bis 13, dadurch gekennzeichnet, daß man in der Fettsäuren und gegebenenfalls Zinkseifen enthaltenden Schmelze nacheinander die Fettsäurediamide und Fettsäureester sowie gegebenenfalls die Metallseifen aus der von Mg, Ca, Ba, Cd und Pb gebildeten Gruppe, insbesondere Ca-Seifen, erzeugt.

15. Verfahren nach mindestens einem der Ansprüche 1 bis 14, dadurch gekennzeichnet, daß man der die Fettsäurediamide und die Ester der Fettsäuren mit mehrfunktionellen Alkanolen enthaltenden Schmelze vor der Umsetzung mit den basischen Verbindungen zweiwertiger Metalle weitere C₈-C₂₂-Fettsäuren zusetzt.

16. Verfahren nach mindestens einem der Ansprüche 1 bis 15 zur Herstellung Zinkseifen enthaltender Gemische, dadurch gekennzeichnet, daß man Fettsäuren, Zinkseifen bzw. basische Zinkverbindungen, gegebenenfalls basische Verbindungen anderer zweiwertiger Metalle sowie Diamine und Fettalkohole bzw. mehrfunktionelle Alkanole in molaren Verhältnissen zueinander umsetzt, daß sich Säurezahlen der Gemische im Bereich von 5 bis 20 ergeben.

17. Gemische von hellfarbigen bis(C₈-C₂₂-Fettsäure)alkylendiamiden mit Estern von C₈-C₂₂-Fettsäuren mit Gewichtsverhältnissen von Alkylendiamiden zu Fettsäureestern und gegebenenfalls Metallseifen in Gewichtsverhältnissen zueinander von 4 bis 6 : 1 bis 2 : 2 bis 4, erhältlich nach einem Verfahren gemäß mindestens einem der Ansprüche 1 bis 16.

18. Verwendung von hellfarbige bis(C₈-C₂₂-Fettsäure)-alkylendiamide enthaltenden Gemischen, erhältlich nach einem Verfahren gemäß mindestens einem der Ansprüche 1 bis 16 oder nach Anspruch 17 als Additive für Kunststoffe, insbesondere auf Basis von PVC bzw. Copolymeren derselben.

## Claims

1. A process for the production of mixtures of light-coloured bis-(C₈₋₂₂ fatty acid)-alkylenediamides and C₈₋₂₂ fatty acid esters and, optionally, metal soaps of C₈₋₂₂ fatty acids, characterized in that, in a melt containing C₈₋₂₂ fatty acids, the fatty acids are partly condensed with diamines corresponding to the following general formula:
NH₂-R-NH₂
in which R is a linear, branched or cyclic alkylene group containing 2 to 12 carbon atoms,
to form the corresponding fatty acid diamides, partly esterified with C₈₋₂₂ fatty alcohols and/or polyfunctional alkanols containing 2 to 15 carbon atoms and 2 to 6 hydroxyl groups and, optionally, partly reacted with basic compounds of divalent metals to form the corresponding metal soaps, the condensation of the fatty acids with the diamines being carried out before esterification of the fatty acids with the fatty alcohols and/or polyfunctional alkanols.

2. A process as claimed in claim 1, characterized in that saturated c₁₆₋₂₂ fatty acids or mixtures thereof are used.

3. A process as claimed in claim 1 or 2, characterized in that diamines from the group consisting of ethylenediamine, tetramethylenediamine and hexamethylenediamine are used.

4. A process as claimed in at least one of claims 1 to 3, characterized in that 2.2 to 10 and, more particularly, 2.5 to 4 moles of the fatty acids present in the melt are used per mole of diamine in the condensation reaction.

5. A process as claimed in at least one of claims 1 to 4, characterized in that the fatty acid diamides, fatty acid esters and, optionally, the metal soaps are prepared in the melt in ratios by weight to one another of 4 to 6:1 to 2:2 to 4.

6. A process as claimed in at least one of claims 1 to 5, characterized in that the fatty acids and diamines are condensed at temperatures of 140 to 190°C and, more particularly, at temperatures of 160 to 180°C.

7. A process as claimed in at least one of claims 1 to 6, characterized in that the condensation reaction is terminated when the reaction mixture reaches an amine value of less than 1.

8. A process as claimed in at least one of claims 1 to 7, characterized in that the fatty acids and fatty alcohols or polyfunctional alkanols are reacted in the presence of esterification catalysts at temperatures of the melt of 150 to 190°C.

9. A process as claimed in at least one of claims 1 to 8, characterized in that saturated C₁₂₋₂₂ fatty alcohols are used for the esterification of the fatty acids.

10. A process as claimed in at least one of claims 1 to 9, characterized in that polyfunctional alkanols from the group consisting of ethylene glycol, propylene glycol, glycerol, diglycerol, triglycerol, tetraglycerol, trimethylol propane, ditrimethylol propane, pentaerythritol and dipentaerythritol are used for the esterification and are reacted with the fatty acids to form full or partial esters.

11. A process as claimed in at least one of claims 1 to 10, characterized in that the reaction to form the metal soaps is carried out at temperatures of the melt of 150 to 160°C.

12. A process as claimed in at least one of claims 1 to 11, characterized in that oxides, hydroxides and/or carbonates of one or more metals from the group consisting of Mg, Ca, Ba, Cd, Zn and Pb, more particularly Ca and/or Zn, are used for forming the metal soaps of the fatty acids.

13. A process as claimed in at least one of claims 1 to 12, characterized in that a zinc soap of the fatty acids is added to or produced in the melt containing the fatty acid before condensation and esterification and, optionally, formation of the metal soap.

14. A process as claimed in at least one of claims 1 to 13, characterized in that the fatty acid diamides and fatty acid esters and, optionally, the metal soaps from the group consisting of Mg, Ca, Ba, Cd and Pb, more particularly Ca soaps, are successively produced in the melt containing fatty acids and optionally zinc soaps.

15. A process as claimed in at least one of claims 1 to 14, characterized in that, before the reaction with the basic compounds of divalent metals, other C₈₋₂₂ fatty acids are added to the melt containing the fatty acid diamides and the esters of the fatty acids with polyfunctional alkanols.

16. A process as claimed in at least one of claims 1 to 15 for the production of mixtures containing zinc soaps, characterized in that fatty acids, zinc soaps or basic zinc compounds, optionally basic compounds of other divalent metals and also diamines and fatty alcohols or polyfunctional alkanols are reacted in such molar ratios to one another than acid values of the mixtures of 5 to 20 are obtained.

17. Mixtures of light-coloured bis-(C₈₋₂₂ fatty acid)-alkylenediamides with esters of C₈₋₂₂ fatty acids in ratios by weight of the alkylenediamides to the fatty acid esters and optionally metal soaps of 4 to 6:1 to 2:2 to 4 obtainable by the process claimed in at least one of claims 1 to 16.

18. The use of mixtures containing light-coloured bis-(C₈₋₂₂ fatty acid)-alkylenediamides obtainable by the process claimed in at least one of claims 1 to 16 or claim 17 as additives for plastics, more particularly based on PVC or copolymers thereof.

## Revendications

1. Procédé d'obtention de mélanges de bis (acides gras en C₈-C₂₂) alcoylènediamides de couleur claire et d'esters d'acides gras en C₈ à C₂₂ ainsi que le cas échéant de savons métalliques d'acides gras en C₈ à C₂₂, caractérisé en ce que l'on condense dans un produit de fusion contenant des acides gras ayant de 8 à 22 atomes de carbone une partie des acides gras avec des diamines de formule générale :
NH₂-R-NH₂
dans laquelle R est un groupe alcoylène en chaîne droite, ramifié ou cyclique, ayant de 2 à 12 atomes de carbone
- en diamides d'acide gras correspondants, en ce que l'on estérifie une partie des acides gras avec des alcools gras ayant de 8 à 22 atomes de carbone et/ou des alcanols plurifonctionnels ayant de 2 à 15 atomes de carbone et de 2 à 6 groupes hydroxyle ainsi que le cas échéant on fait réagir une partie des acides gras avec des dérivés basiques de métaux bivalents en savons métalliques correspondants, procédé dans lequel on effectue la condensation des acides gras avec les diamines avant l'estérification des acides gras avec les alcools gras et/ou les alcanols plurifonctionnels.

2. Procédé selon la revendication 1, caractérisé en ce que l'on utilise des acides gras saturés ayant de 16 à 22 atomes de carbone ou des mélanges de ceux-ci.

3. Procédé selon la revendication 1 ou la revendication 2, caractérisé en ce que l'on utilise des diamines choisies dans le groupe formé de l'éthylènediamine, la tétraméthylènediamine et l'hexaméthylènediamine.

4. Procédé selon au moins une des revendications 1 à 3, caractérisé en ce que l'on met en oeuvre pour la condensation de 2,2 à 10, en particulier de 2,5 à 4 mol d'acides gras contenus dans le produit de fusion par mol de diamine.

5. Procédé selon au moins une des revendications 1 à 4, caractérisé en ce que l'on produit dans le produit de fusion les diamides d'acide gras, les esters d'acide gras, et le cas échéant les savons métalliques dans les rapports pondéraux les uns par rapport aux autres de 4 à 6 : 1 à 2 : 2 à 4.

6. Procédé selon au moins une des revendications 1 à 5, caractérisé en ce que l'on condense les acides gras et les diamines à des températures allant de 140 à 190°C, en particulier de 160 à 180°C

7. Procédé selon au moins une des revendications 1 à 6, caractérisé en ce que l'on achève la condensation une fois qu'on atteint un indice d'amine du mélange réactionnel de moins de 1.

8. Procédé selon au moins une des revendications 1 à 7, caractérisé en ce que l'on fait réagir les acides gras et les alcools gras ou les alcanols plurifonctionnels à des températures du produit de fusion allant de 150 à 190°C en présence de catalyseurs d'estérification;

9. Procédé selon au moins une des revendications 1 à a, caractérisé en ce que l'on utilise pour l'estérification des acides gras, des alcools gras saturés ayant de 12 à 22 atomes de carbone.

10. Procédé selon au moins une des revendications 1 à 9, caractérisé en ce que l'on utilise pour l'estérification des alcanols plurifonctionnels choisis dans le groupe formé de l'éthylèneglycol, du propylèneglycol. du glycérol, du diglycérol, du triglycérol, du tétraglycérol, du triméthylolpropane, du di-triméthylolpropane, du pentaerythritol, et du di-pentaérythritol et on fait réagir ceux-ci avec des acides gras en esters complets ou partiels.

11. Procédé selon au moins une des revendications 1 à 10 caractérisé en ce que l'on entreprend la réaction en savons métalliques à des températures de produit de fusion allant de 150 à 160°C.

12. Procédé selon au moins une des revendications 1 à 11, caractérisé en ce que l'on utilise pour la formation des savons métalliques d'acides gras, des oxydes, des hydroxydes et/ou des carbonates d'un ou plusieurs métaux choisis dans le groupe formé du Mg, Ca, Ba, Cd, Zn, et Pb, en particulier du Ca et/ou du Zn.

13. Procédé selon au moins une des revendications 1 à 12, caractérisé en ce que l'on ajoute, au produit de fusion contenant les acides gras, un savon de zinc d'acides gras avant la condensation et l'estérification ainsi que le cas échéant avant la formation de savon métallique ou bien on le produit dans le produit de fusion.

14. Procédé selon au moins une des revendications 1 à 13, caractérisé en ce que l'on produit dans le produit de fusion contenant les acides gras et, le cas échéant, des savons de zinc, l'un après l'autre les diamides d'acide gras et les esters d'acide gras ainsi que le cas échéant les savons métalliques choisis dans le groupe formé de Mg, Ca, Ba, Cd et Pd, en particulier les savons de Ca.

15. Procédé selon au moins une des revendications 1 à 14, caractérisé en ce que l'on ajoute au produit de fusion contenant les diamides d'acide gras et les esters d'acides gras avec des alcanols plurifonctionnels avant la réaction avec les dérivés basiques, des métaux bivalents d'autres acides gras en C₈ à C₂₂..

16. Procédé selon au moins une des revendications 1 à 15 pour l'obtention de mélanges contenant des savons de zinc, caractérisé en ce que l'on fait réagir des acides gras, des savons de zinc ou des dérivés basiques du zinc, le cas échéant des dérivés basiques d'autres métaux bivalents, ainsi que des diamines et des alcools gras ou des alcanols plurifonctionnels dans les rapports molaires l'un par rapport à l'autre, tels qu'on obtienne des indices d'acidité des mélanges dans la zone de 5 à 20.

17. Mélanges de bis (acides gras en C₈ à C₂₂) alcoylènediamides de couleur claire avec des esters d'acides gras en C₈ à C₂₂ ayant des rapports pondéraux d'alcoylènediamides aux esters d'acides gras et le cas échéant aux savons métalliques dans des rapports pondéraux les uns par rapport aux autres allant de 4 à 6 : 1 à 2 : 2 à 4, accessibles selon le procédé conforme à au moins une des revendications 1 à 16.

18. Utilisation des mélanges contenant des (bis acides gras en C₈ à C₂₂) alcoylènediamides, accessibles selon un procédé conformément à au moins une des revendications 1 à 16, ou selon la revendication 17, en tant qu'additifs pour matières plastiques, en particulier à base de PVC ou de copolymères de celles-ci.
